Europäisches Patentamt

European Patent Office

Office européen des brevets

(11) Veröffentlichungsnummer : **0 062 027**
**B1**

## EUROPÄISCHE PATENTSCHRIFT

(45) Veröffentlichungstag der Patentschrift :
18.09.85

(51) Int. Cl.⁴ : **C 13 K   1/02, C 12 P 19/02**

(21) Anmeldenummer : 82890041.5

(22) Anmeldetag : 18.03.82

(54) **Verfahren zur Gewinnung von Monosacchariden sowie Anlage zur Durchführung des Verfahrens.**

(30) Priorität : 25.03.81 AT 1394/81

(43) Veröffentlichungstag der Anmeldung :
06.10.82 Patentblatt 82/40

(45) Bekanntmachung des Hinweises auf die Patenterteilung : 18.09.85 Patentblatt 85/38

(84) Benannte Vertragsstaaten :
BE CH DE FR GB IT LI LU NL SE

(56) Entgegenhaltungen :
US-A- 3 764 475
US-A- 3 972 775
BIOTECHNOLOGY AND BIOENGINEERING, Band 23,
Nr. 1, Januar 1981, NEW YORK (US), C.R. WILKE et
al.: "Raw materials evaluation and process development studies for conversion of biomass to sugars and
ethanol", Seiten 163-183

(73) Patentinhaber : STEYRERMÜHL Papierfabriks- und
Verlags-Aktiengesellschaft
Fabriksplatz 1
A-4662 Steyrermühl (AT)

(72) Erfinder : Meindl, Norbert, Dr. Dipl.-Ing.
Lannastrasse 11
AT-4810 Gmunden (AT)
Erfinder : Schwarzl, Karl, Dr.
Villenstrasse 19
AT-4662 Steyrermühl (AT)

(74) Vertreter : Wolfram, Gustav, Dipl.-Ing.
Schwindgasse 7 P.O. Box 205
A-1041 Wien (AT)

## Beschreibung

Die Erfindung betrifft ein Verfahren zur Gewinnung von Monosacchariden aus cellulosehältigem Ausgangsmaterial durch Spaltung mittels Cellulase sowie gegebenenfalls von durch Vergären der Monosaccharide erhaltenen Produkten, wobei das zerkleinerte cellulosehältige Ausgangsmaterial mit cellulasehältiger, wässeriger Lösung bei einer Temperatur zwischen Raumtemperatur und etwa 50 °C unter Atmosphärendruck umgesetzt wird, sowie eine Anlage zur Durchführung des Verfahrens, umfassend eine Zerkleinerungsvorrichtung für cellulosehältiges Ausgangsmaterial, eine Sterilisationseinheit für das zerkleinerte Ausgangsmaterial, einen Mischer zum Vermengen des sterilisierten, zerkleinerten Materials mit aus einer Leitung zuführbarer Enzymlösung sowie gegebenenfalls einen Gärbehälter und eine Destillations- bzw. Rektifikationsvorrichtung.

Verfahren zur enzymatischen Herstellung von Zucker aus Cellulose sind aus den US-A 3,972,775 und 4,097,333 bekannt, wobei in der US-A 3,972,775 ein Verfahren beschrieben ist, welches sowohl die Herstellung des Enzyms durch Züchten der Mikroorganismen als auch die Hydrolyse cellulosischen Materials mittels des hergestellten Enzyms umfaßt. Die Hydrolyse bzw. Spaltung der Cellulose wird einstufig durchgeführt. Gemäß der US-A 4,097,333 wird cellulosisches Material mit Äthylen in Kontakt gebracht, um die Glucoseausbeute bei der enzymatischen Hydrolyse zu erhöhen.

Eine Arbeit von C.R. Wilke et al. in Biotechnology and Bioengineering, Band 23, Nr. 1, Jänner 1981, New York, Seiten 163 bis 183 beschäftigt sich mit der Aufarbeitung von cellulosehältigem Material unter Gewinnung von Zucker und Äthanol. Dabei wird beispielsweise Maisstrohhäcksel zunächst einer Säurebehandlung unterworfen, wobei Pentosen extrahiert werden. Die verbleibenden Feststoffe werden abgetrennt, gewaschen und sodann im Gegenstrom mit einer enzym- und zuckerhältigen Lösung aus fünf gerührten, zu einer Hydrolysator-Einheit zusammengefaßten Digestern in Kontakt gebracht, wobei das gelöste Enzym an den Feststoffen adsorbiert wird. Die so vorbehandelten Feststoffe gelangen sodann als wässerige Suspension mit nur 5 Massen % Feststoffen in den Hydrolysator, die nach Abtrennung der restlichen Feststoffe resultierende enzymhältige Zuckerlösung kann teilweise in den Hydrolysator rückgeführt werden, der Rest der Lösung wird nach der beschriebenen Adsorption des Enzyms an dem extrahierten festen Ausgangsmaterial unter Vakuum eingedampft. Die erhaltene, an Zucker aufkonzentrierte Lösung wird einer alkoholischen Gärung unterworfen. Das celluloseabbauende Enzym wird durch Kultivieren von Trichoderma viride QM 9414 in einem zweistufigen Fermentationssystem erzeugt und in wässeriger Lösung direkt dem Hydrolysator zugesetzt.

Die Schwierigkeit einer technischen Anwendung des enzymatischen Abbaus zur Gewinnung von Monosacchariden und gegebenenfalls von Gärprodukten — wie Alkohole und Protein — aus diesen Kohlenhydraten besteht hauptsächlich darin, daß die Konzentration an Zuckern bzw. an Gärprodukten, die bei mikrobiologischer Spaltung gewonnen werden, sehr gering ist — beispielsweise werden nach 48-stündiger Einwirkzeit von Cellulase maximale Hydrolyseraten in der Größenordnung von wenigen Prozenten des Ausgangsmaterials erzielt — mit der Folge, daß die Konzentrierung bzw. Reingewinnung mit weitaus zu hohen Energiekosten belastet ist. Bei der technischen Gewinnung von Zuckern und in weiterer Folge von Alkohol aus cellulosehältigem Ausgangsmaterial wurden deshalb bisher Spaltverfahren mittels Säuren, meist unter Anwendung hoher Drucke und Temperaturen, bevorzugt. Diese Verfahren sind gleichfalls sehr energieaufwendig, zusätzlich ergeben sich Probleme hinsichtlich der Rückgewinnung der Säuren.

Die Erfindung bezweckt die Vermeidung dieser Nachteile und Schwierigkeiten und stellt sich die Aufgabe, ein kontinuierliches technisches Verfahren der eingangs definierten Art mit besserer Verwertung des cellulosischen Rohmaterials zu schaffen, welches Verfahren bei Raumtemperatur oder schwach erhöhter Temperatur vor sich geht, einen verhältnismäßig geringen Energieaufwand erfordert, den Einsatz von Hochdruckapparaten vermeidet, mit billigen Ausgangs- bzw. Abfallstoffen arbeitet und die Umwelt nicht belastet.

Die gestellte Aufgabe wird dadurch gelöst, daß die Reaktion in einer Mehrzahl von Reaktionsstufen durchgeführt wird und nach jeder Reaktionsstufe die enzym- und zuckerhältige Lösung von den Feststoffen abgetrennt wird, die Feststoffe nach Auswaschen der gebildeten Kohlenhydrate jeweils der nächsten Reaktionsstufe zugeführt werden, die aus den Reaktionsstufen erhaltenen enzym- und zuckerhältigen Lösungen gesammelt und gegebenenfalls durch Zusatz von zuckerverwertenden Mikroorganismen vergoren werden.

Die enzymatische Hydrolyse wird somit mehrstufig durchgeführt, u. zw. mindestens zwei-, vorzugsweise jedoch bis sechsstufig. Das zu hydrolysierende Material wird durch sämtliche hintereinander geschaltete Reaktionsstufen geführt, wobei zwischen den einzelnen Stufen jeweils eine Auswaschung der gebildeten Kohlenhydrate stattfindet. Dadurch wird eine Hemmung der Hydrolyse, die durch eine zu hohe Zuckerkonzentration bewirkt wird, vermieden und es werden somit erfindungsgemäß eine Beschleunigung sowie ein besserer Ausnützungsgrad gegenüber bekannten Verfahren erzielt. Nach der letzten Reaktionsstufe wird der nicht hydrolysierte Anteil der Feststoffe von der Lösung abgetrennt, ausgewaschen, einer Verbrennungsanlage zugeführt oder anderweitig verwendet.

Das cellulosehältige Ausgangsmaterial wird zweckmäßig sterilisiert, bevor es mit der cellulasehältigen, wässerigen Lösung umgesetzt wird. Die Sterilisation kann durch Ozonisieren, Zugabe eines Biocids, wie Pentachlorphenol-Natrium oder Einleiten von Dampf erfolgen.

Vorteilhaft wird vor dem Abtrennen der Feststoffe aus der enzym- und zuckerhältigen Lösung die aus den Reaktionsstufen erhaltene Suspension breifein gemahlen. Durch diese Mahlung zwischen den Reaktionsstufen werden neue Oberflächen des cellulosehältigen Materials für den Angriff des Enzyms geschaffen, außerdem können entstandene, noch oligomere Zucker besser ausgewaschen werden.

Den anschließend abgetrennten Feststoffen wird vorzugsweise vor Zuführung in die nächste Reaktionsstufe frische Cellulaselösung zugemischt, sofern sie nicht aus der letzten Reaktionsstufe stammen. Der Vorteil dieser Ausführungsform besteht darin, daß die Hydrolyse beschleunigt wird.

Zweckmäßig wird die aus den Reaktionsstufen erhaltene und von den Feststoffen abgetrennte enzym- und zuckerhältige Lösung mit immobilisiertem Enzym vollständig zu Monosacchariden abgebaut, wobei als immobilisiertes Enzym insbesondere immobilisierte β-Glucosidase eingesetzt wird. Bei immobilisierten Enzymen liegen die Enzyme an einen Träger, beispielsweise an Polyacrylamidgel, gebunden vor, so daß sie bei voller Aktivität nicht von einer mit ihnen in Kontakt kommenden Lösung ausgewaschen werden können.

Nach einer bevorzugten Ausführungsform des erfindungsgemäßen Verfahrens werden zur Gewinnung von Monosacchariden die enzym- und zuckerhältigen Lösungen teilweise als Verdünnung in die einzelnen Reaktionsstufen rückgeführt, mit den Umsetzungsprodukten aus denselben wieder abgezogen und teilweise mit frischem cellulosehältigem Material vermischt, wobei das Enzym an dem cellulosehältigen Material adsorbiert und auf diese Weise abgetrennt bzw. rückgewonnen wird. Einer der Hauptbestandteile der erhaltenen zuckerhältigen Lösung ist Glucose, deren Konzentration auf diese Weise erhöht werden kann. Die enzym- und zuckerhältigen Lösungen sind darüber hinaus cellobiosefrei und der rückgeführte Teil hemmt daher nicht die Hydrolyse. Die von Enzym befreite Zuckerlösung kann einer weiteren Verwertung zugeführt werden. Das cellulosehältige Material mit daran adsorbiertem Enzym wird als Ausgangsstoff wieder in das Verfahren rückgeführt.

Bei einer vorteilhaften Ausgestaltung des erfindungsgemäßen Verfahrens zur Gewinnung von Gärprodukten, wie Äthanol, werden nach Vergären der enzym- und zuckerhältigen Lösungen die Mikroorganismen von der gärprodukthältigen Flüssigkeit abgetrennt, die gärprodukthältige Flüssigkeit wird teilweise als Verdünnung in die einzelnen Reaktionsstufen rückgeführt, mit den Umsetzungsprodukten aus denselben wieder abgezogen und teilweise mit frischem cellulosehältigem Material vermischt, wobei das Enzym an dem cellulosehältigen Material adsorbiert und auf diese Weise abgetrennt bzw. rückgeonnen wird, und die Gärprodukte werden aus der gärprodukthältigen Flüssigkeit durch Destillation bzw. Rektifikation in reiner Form gewonnen.

Bei dieser Ausgestaltung braucht die Cellulase vor der Vergärung nicht von der Zuckerlösung abgetrennt zu werden. Das Verfahren kann zur Gewinnung von Proteinen auch so geführt werden, daß hauptsächlich Biomasse, d. h. beispielsweise Hefezellen, Pilzmycel und sonstige Mikroorganismen, gebildet wird und die Entstehung anderer Gärprodukte mehr oder weniger in den Hintergrund tritt.

Die zur Vergärung eingesetzten Mikroorganismen werden beispielsweise durch Zentrifugieren abgetrennt und rückgeführt bzw. aus dem System ausgeschleust. Die so erhaltene Biomasse kann als Viehfutterzusatz dienen.

Der Feststoffanteil kann in den einzelnen Reaktionsstufen zweckmäßig auf etwa 10 % Masse gehalten werden. Durch die teilweise Rückführung der enzym- und zuckerhältigen Lösungen bzw. der gärprodukthältigen Flüssigkeit in die hintereinander angeordneten Reaktionsstufen nimmt in diesen die Konzentration an Zuckern bzw. Gärprodukten jeweils zu. Durch das Rückführen vergorener Flüssigkeit kann die Glucosekonzentration in den einzelnen Reaktionsstufen niedrig gehalten werden. Auf diese Weise wird die ab einer bestimmten Konzentration an Glucose erfolgende Hemmung der enzymatischen Hydrolyse verhindert. Darüber hinaus wirkt sich ein gewisser Äthanolanteil im Hydrolysenteil günstig aus, weil ein eventuelles Wachstum unerwünschter Mikroorganismen dadurch weitgehend unterbunden wird. Vorteilhaft werden die enzym- und zuckerhältigen Lösungen bzw. die gärprodukthältige Flüssigkeit durch einen Bodeneinlaß in die Behälter der einzelnen Reaktionsstufen rückgeführt, da eine Suspension von cellulosehältigem Ausgangsmaterial, wie Holz, Stroh und Altpapier, mit einem Feststoffanteil von etwa 10 % schwer pumpbar ist und die Austragung des Feststoffes aus den einzelnen Reaktionsstufen auf diese Art erheblich erleichtert wird.

Eine Anlage der eingangs beschriebenen Art zur Durchführung des erfindungsgemäßen Verfahrens ist dadurch gekennzeichnet, daß jeder von wenigstens zwei Hydrolysierbehältern über eine Leitung einerseits mit dem Mischer und anderseits mit dem Feststoffabzug einer Trennvorrichtung in Verbindung bringbar ist, daß der Bodenteil jedes Hydrolysierbehälters über eine Suspensions-Sammelleitung mit der Trennvorrichtung verbindbar ist, wobei gegebenenfalls zwischen die Sammelleitung und die Trennvorrichtung der Reihe nach ein Rührwerksbehälter und eine Mühle geschaltet sind und daß der Auslaß für die flüssigen Anteile aus der Trennvorrichtung über eine Leitung und eine Zuführung mit jedem Hydrolysierbehälter sowie mit dem Gärbehälter verbindbar ist.

Die Erfindung wird im folgenden anhand eines Fließschemas und eines Beispieles näher erläutert.

. Cellulosehältiges Ausgangsmaterial wird zunächst einer Zerkleinerungsvorrichtung 1 und anschließend einer Zwei-Walzen-Mühle 2 zugeführt. Aus der Mühle tritt das Material in einen Mischer 3 ein, welcher mit einer Zuführung 4 für Sterilisationsmittel ausgestattet ist. Das mit dem Sterilisationsmittel versetzte Material gelangt in einen Sterilisationsbehälter 5, in welchem es eine vorgegebene Zeitspanne verweilt. Aus diesem Behälter wird das sterilisierte Material über eine Förderleitung 6 einem weiteren Mischer 7 zugeführt, in welchem es mit cellulasehältiger, wässeriger Lösung aus der Leitung 8 versetzt wird. Von dem Mischer 7 aus werden die einzelnen Reaktionsstufen bzw. Hydrolysierbehälter 9 über die Leitung 10 beschickt. Eine Sammelleitung 11 dient zum Abführen der Suspension aus den einzelnen Hydrolysierbehältern 9 in einen Rührwerksbehälter 12, welcher weiters Zuführungen für ozonisiertes Frischwasser (13), für Abwasser (14) sowie für rückgeführte enzym- und zuckerhältige Lösungen bzw. gärprodukthältige Flüssigkeiten (15) aufweist. Aus dem Behälter 12 gelangt die Suspension in eine Mühle 16, in welcher sie breifein gemahlen wird. Im Anschluß daran wird die Lösung von den Feststoffen mittels einer Zentrifuge 17 abgetrennt. Die Feststoffe werden nach Vorwärmung in einem Wärmeaustauscher 18 über die Leitung 10 jeweils dem nächsten Hydrolysierbehälter 9 der Reihe zugeführt, nachdem sie einen weiteren Mischer 19 durchlaufen haben, in welchem sie über die Leitung 8 gegebenenfalls mit frischer Cellulaselösung vermengt werden. Die im letzten Hydrolysierbehälter der Reihe noch verbliebenen Feststoffe werden durch die Abführung 20 entfernt und beispielsweise verbrannt. Um die Kohlenhydrat- bzw. Alkoholverluste zu verringern, kann das Material aus dem letzten Hydrolysierbehälter durch die Leitung 20′ nochmals dem Rührwerksbehälter 12 zugeführt und dort mit Frischwasser gewaschen werden.

Die flüssigen Anteile aus der Zentrifuge 17 werden durch einen mit immobilisiertem Enzym gefüllten Reaktor 21 geleitet und dann über die Leitungen 22 und 15 teilweise als Verdünnung in die einzelnen Hydrolysierbehälter oder in den Rührwerksbehälter 12 rückgeführt bzw. über die Leitung 15 in einen Rührwerksbehälter 23 gebracht, welcher über eine Zuführung 24 mit zerkleinertem cellulosehältigem Material aus dem Sterilisationsbehälter 5 beaufschlagt werden kann. Zur Trennung des Feststoffanteiles von der Flüssigkeit ist eine Zentrifuge 25 vorgesehen, aus der das mit Cellulase beladene feste Material dem Mischer 7 zugesetzt wird und die erhaltene enzymfreie Zuckerlösung über die Leitung 26 aus der Anlage abgeführt wird.

Alternativ können die flüssigen Anteile aus der Zentrifuge 17 nach ihren Austreten aus dem Reaktor 21 in einen mit einer Zuführung 27 für Mikroorganismen ausgestatteten Gärbehälter 28 gebracht werden. Nach einer bestimmten Verweilzeit wird das Gärprodukt in eine Trennvorrichtung, in der dargestellten Anlage beispielsweise eine Zentrifuge 29, geleitet, wo eine Trennung in Biomasse und gärprodukthältige Flüssigkeit stattfindet. Die Biomasse kann durch die Zuführung 27 wieder in den Gärbehälter 28 rückgeführt werden oder über die Leitung 30 aus der Anlage ausgeschleust werden. Die gärprodukthältige Flüssigkeit gelangt zunächst in einen Zwischenbehälter 31, aus dem sie mittels einer Pumpe 32 teilweise über die Leitung 15 als Verdünnung in die einzelnen Hydrolysierbehälter 9 oder in den Rührwerksbehälter 12 rückgeführt bzw. zur Rückgewinnung des Enzyms in den Rührwerksbehälter 23 eingebracht wird. Die enzymfreie gärprodukthältige Flüssigkeit aus der Zentrifuge 25 wird einer Destillations- bzw. Rektifikationseinheit 33 zugeführt, mittels welcher die Auftrennung dieser Flüssigkeit in Gärprodukte, wie z. B. Äthanol, Fuselöle, Glycerin vorgenommen wird. Die Gärprodukte werden über Leitungen 34, 35 abgezogen, das resultierende Abwasser wird entweder in den Mischer 7 oder über die Zuführung 14 in den Rührwerksbehälter 12 als Verdünnung geleitet oder aber es verläßt die Anlage über die Leitung 36.

Beispiel

Altpapier, welches 1 000 kg Trockensubstanz enthielt, wurde zerkleinert, 20 min lang bei 120°C sterilisiert, mit 8 950 l Wasser versetzt, auf einen pH-Wert von 4,8 eingestellt und mit 50 l Enzymsuspension, welche $10^7$ FPU an Cellulase enthielt, vermischt (FPU : Filterpapiereinheiten, früher FPD-activity-units ; nach N. Toyama, Advances in Enzyme Hydrolysis of Cellulose and Related Materials, E.T. Reese, Ed. Pergamon Press, London 1963, Seite 235). Der Feststoffanteil der Suspension betrug somit etwa 10 % Masse. Die gesamte Suspension wurde durch 6 Reaktionsstufen bzw. Hydrolysierbehälter geführt, wobei die Verweilzeit jeweils 8 Stunden betrug.

Zwischen den einzelnen Hyrolysierstufen wurden entstandene Kohlenhydrate ausgewaschen und die Lösungen gesammelt. Zum Auswaschen wurden die Lösungen jeweils rückgeführt, abschließend wurde mit Frischwasser gewaschen. In den gesammelten Lösungen mit einem Volumen von 15 000 l befanden sich 323 kg reduzierende Zucker, davon 221 kg Glucose. Weiters war noch Mannose und Xylose vorhanden.

Bei der Vergärung der Zucker mittels Hefe entstanden daraus 109 kg Äthanol. In der Lösung waren weiters noch $7.10^6$ FPU Enzym vorhanden. Der nichthydrolysierte Anteil an Altpapier betrug 677 kg.

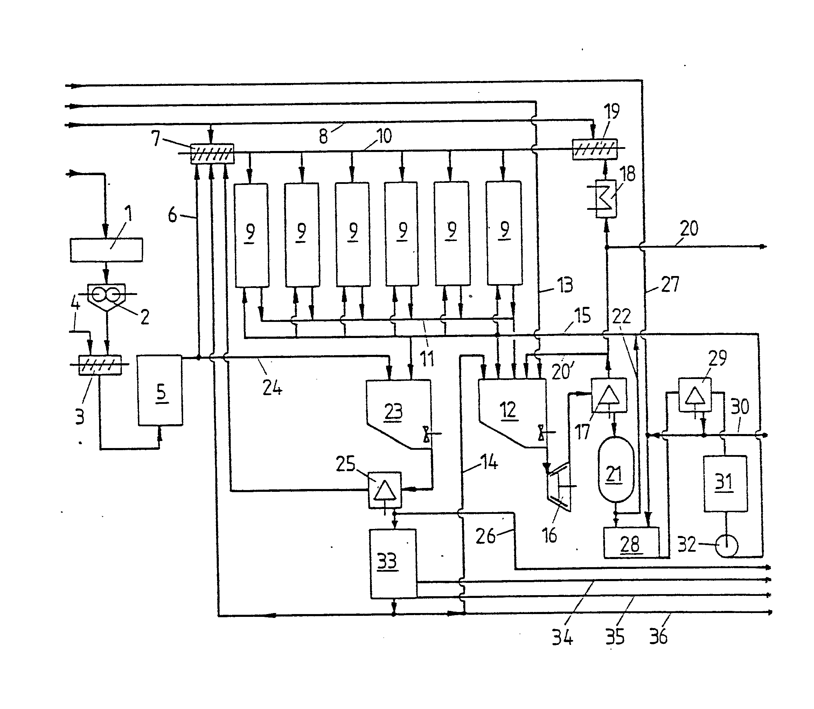

**Patentansprüche**

1. Verfahren zur Gewinnung von Monosacchariden aus cellulosehältigem Ausgangsmaterial durch Spaltung mittels Cellulase sowie gegebenenfalls von durch Vergären der Monosaccharide erhaltenen Produkten, wobei das zerkleinerte cellulosehältige Ausgangsmaterial mit cellulasehältiger, wässeriger Lösung bei einer Temperatur zwischen Raumtemperatur und etwa 50 °C unter Atmosphärendruck umgesetzt wird, dadurch gekennzeichnet, daß die Reaktion in einer Mehrzahl von Reaktionsstufen durchgeführt wird und nach jeder Reaktionsstufe die enzym- und zuckerhältige Lösung von den Feststoffen abgetrennt wird, die Feststoffe nach Auswaschen der gebildeten Kohlenhydrate jeweils der nächsten Reaktionsstufe zugeführt werden, die aus den Reaktionsstufen erhaltenen enzym- und zuckerhältigen Lösungen gesammelt und gegebenenfalls durch Zusatz von zuckerverwertenden Mikroorganismen vergoren werden.

2. Verfahren nach Anspruch 1, dadurch gekennzeichnet, daß das cellulosehältige Ausgangsmaterial sterilisiert wird, bevor es mit der cellulasehältigen, wässerigen Lösung umgesetzt wird.

3. Verfahren nach den Ansprüchen 1 und 2, dadurch gekennzeichnet, daß vor dem Abtrennen der Feststoffe aus der enzym- und zuckerhältigen Lösung die aus den Reaktionsstufen erhaltene Suspension breifein gemahlen wird.

4. Verfahren nach den Ansprüchen 1 bis 3, dadurch gekennzeichnet, daß den Feststoffen vor Zuführung in die nächste Reaktionsstufe frische Cellulaselösung zugemischt wird.

5. Verfahren nach den Ansprüchen 1 bis 4, dadurch gekennzeichnet, daß die aus den Reaktionsstufen erhaltene und von den Feststoffen abgetrennte enzym- und zuckerhältige Lösung mit immobilisiertem Enzym vollständig zu Monosacchariden abgebaut wird.

6. Verfahren nach Anspruch 5, dadurch gekennzeichnet, daß als immobilisiertes Enzym immobilisierte β-Glucosidase eingesetzt wird.

7. Verfahren nach den Ansprüchen 1 bis 6 zur Gewinnung von Monosacchariden, dadurch gekennzeichnet, daß die enzym- und zuckerhältigen Lösungen teilweise als Verdünnung in die einzelnen Reaktionsstufen rückgeführt, mit den Umsetzungsprodukten aus denselben wieder abgezogen und teilweise mit frischem cellulosehältigem Material vermischt werden, wobei das Enzym an dem cellulosehältigen Material adsorbiert und auf diese Weise abgetrennt bzw. rückgewonnen wird.

8. Verfahren nach den Ansprüchen 1 bis 6 zur Gewinnung von Gärprodukten, dadurch gekennzeichnet, daß nach Vergären der enzym- und zuckerhältigen Lösungen die Mikroorganismen von der gärprodukthältigen Flüssigkeit abgetrennt werden, die gärprodukthältige Flüssigkeit teilweise als Verdünnung in die einzelnen Reaktionsstufen rückgeführt, mit den Umsetzungsprodukten aus denselben wieder abgezogen und teilweise mit frischem cellulosehältigem Material vermischt wird, wobei das Enzym an dem cellulosehältigen Material adsorbiert und auf diese Weise abgetrennt bzw. rückgewonnen wird, und daß die Gärprodukte aus der gärprodukthältigen Flüssigkeit durch Destillation bzw. Rektifikation in reiner Form gewonnen werden.

9. Verfahren nach den Ansprüchen 7 und 8, dadurch gekennzeichnet, daß die enzym- und zuckerhältigen Lösungen bzw. die gärprodukthältige Flüssigkeit durch einen Bodeneinlaß in die Behälter der einzelnen Reaktionsstufen rückgeführt werden.

10. Anlage zur Durchführung des Verfahrens nach den Ansprüchen 1 bis 9, umfassend eine Zerkleinerungsvorrichtung (1) für cellulosehältiges Ausgangsmaterial, eine Sterilisationseinheit (3, 5) für das zerkleinerte Ausgangsmaterial, einen Mischer (7) zum Vermengen des sterilisierten, zerkleinerten Materials mit aus einer Leitung (8) zuführbarer Enzymlösung sowie gegebenenfalls einen Gärbehälter (28) und eine Destillations-bzw. Rektifikationsvorrichtung (33), dadurch gekennzeichnet, daß jeder von wenigstens zwei Hydrolysierbehältern (9) über eine Leitung (10) einerseits mit dem Mischer (7) und anderseits mit dem Feststoffabzug einer Trennvorrichtung (17) in Verbindung bringbar ist, daß der Bodenteil jedes Hydrolysierbehälters (9) über eine Suspensions-Sammelleitung (11) mit der Trennvorrichtung (17) verbindbar ist, wobei gegebenenfalls zwischen die Sammelleitung (11) und die Trennvorrichtung (17) der Reihe nach eine Rührwerksbehälter (12) und eine Mühle (16) geschaltet sind und daß der Auslaß für die flüssigen Anteile aus der Trennvorrichtung (17) über eine Leitung (22) und eine Zuführung (15) mit jedem Hydrolysierbehälter (9) sowie mit dem Gärbehälter (28) verbindbar ist.

## Claims

1. A method for recovering monosaccharides from cellulose-containing starting material by hydrolyzation by means of cellulase as well as, if desired, products obtained by fermentation of the monosaccharides, the disintegrated cellulose-containing starting material being reacted with a cellulase-containing aqueous solution at a temperature of between room temperature and about 50 °C under atmospheric pressure, characterised in that the reaction is carried out in a plurality of reaction stages and the enzyme- and sugar-containing solution is separated from the solids after each reaction stage, the solids are each supplied to the subsequent reaction stage after washing out the carbohydrates formed, the enzyme- and sugar-containing solutions obtained from the reaction stages are collected and, if desired, are fermented by the addition of sugar-consuming microorganisms.

2. A method according to claim 1, characterised in that the cellulose-containing starting material is sterilized prior to being reacted with the cellulase-containing aqueous solution.

3. A method according to claims 1 and 2, characterised in that, prior to the separation of solids from the enzyme- and sugar-containing solution, the suspension obtained from the reaction stages is ground to a pulp.

4. A method according to claims 1 to 3, characterised in that fresh cellulase solution is admixed to the solids prior to supplying them to the subsequent reaction stage.

5. A method according to claims 1 to 4, characterised in that the enzyme- and sugar-containing solution obtained from the reaction stages and separated from the solids is completely degraded to monosaccharides by immobilised enzyme.

6. A method according to claim 5, characterised in that immobilised β-glucosidase is used as the immobilised enzyme.

7. A method according to claims 1 to 6 for recovering monosaccharides, characterised in that the enzyme- and sugar-containing solutions partially are recycled as diluents to the individual reaction stages, are again drawn off the same together with the reaction products and are partially mixed with fresh cellulose-containing material, the enzyme being absorbed on the cellulose-containing material and thus separated and regained.

8. A method according to claims 1 to 6 for recovering fermentation products, characterised in that the micro-organisms are separated from the fermentation-product containing liquor after fermentation of the enzyme- and sugar-containing solutions, the fermentation-product containing liquor partially is recycled as diluent to the individual reaction stages, again drawn off the same together with the reaction products and partially mixed with fresh cellulose-containing material, the enzyme being absorbed on the cellulose-containing material and thus separated and regained, and that the fermentation products are recovered from the fermentation-product containing liquor in pure form by distillation or rectification.

9. A method according to claims 7 and 8, characterised in that the enzyme- and sugar-containing solutions, or fermentation-product containing liquor, are recycled into the vessels of the individual reaction stages through a bottom inlet.

10. A plant for carrying out the method according to claims 1 to 9, comprising an integrating means (1) for cellulose-containing starting material, a sterilisation unit (3, 5) for the integrated starting material, a mixer (7) for mixing the sterilised integrated material with enzymatic solution to be supplied from a duct (8), as well as, if desired, a fermentation vessel (28) and a distillation or rectification means (33), characterised in that each of at least two hydrolyzing vessels (9) are connectable via a duct (10) with the mixer (7) on the one hand and with the solids discharge of a separating means (17) on the other hand, that the bottom part of each hydrolyzing vessel (9) is connectable with the separating means (17) via a suspension-collecting duct (11), an agitation vessel (12) and a mill (16) being disposed in order between the collecting duct (11) and the separating means (17), if desired, and that the outlet for the liquid portions from the separating means (17) is connectable with each hydrolyzing vessel (9) as well as with the fermentation vessel (28) via a duct (22) and a supply (15).

**Revendications**

1. Procédé pour l'obtention de monosaccharides à partir d'une matière première cellulosique par scission à l'aide de cellulase et, le cas échéant, de produits obtenus par fermentation des monosaccharides, dans lequel la matière première cellulosique broyée est mise à réagir avec une solution aqueuse contenant de la cellulase à une température comprise entre la température ambiante et 50 °C environ à pression atmosphérique, caractérisé en ce que la réaction est effectuée dans des stades de réaction multiples, avec séparation de la solution contenant l'enzyme et les sucres d'avec les matières solides après chaque stade de réaction, envoi des matières solides après extraction par lavage des hydrates de carbone formés dans chaque cas au stade de réaction qui suit immédiatement, récolte des solutions contenant l'enzyme et les sucres, obtenues dans les stades de réaction et, le cas échéant, fermentation par addition de micro-organismes utilisant les sucres.

2. Procédé selon la revendication 1, caractérisé en ce que la matière première cellulosique est stérilisée avant d'être mise à réagir avec la solution aqueuse contenant de la cellulase.

3. Procédé selon les revendications 1 et 2, caractérisé en ce que, avant séparation des matières solides et de la solution contenant l'enzyme et les sucres, on broie la suspension obtenue dans les stades de réaction à la finesse d'une bouillie.

4. Procédé selon les revendications 1 à 3, caractérisé en ce que l'on mélange aux matières solides, avant de les envoyer au stade de réaction suivant, de la solution fraîche de cellulase.

5. Procédé selon les revendications 1 à 4, caractérisé en ce que la solution contenant l'enzyme et les sucres obtenue dans les stades de réaction et débarrassée des matières solides est dégradée totalement en monosaccharides à l'aide d'une enzyme immobilisée.

6. Procédé selon la revendication 5, caractérisé en ce que l'on utilise en tant qu'enzyme immobilisée de la β-glucosidase immobilisée.

7. Procédé selon les revendications 1 à 6 pour l'obtention de monosaccharides, caractérisé en ce que les solutions contenant l'enzyme et les sucres sont recyclées en partie en tant que diluants dans les divers stades de réaction, évacuées à nouveau de ceux-ci avec les produits de réaction et mélangées en partie avec de la matière cellulosique fraîche, l'enzyme étant absorbée sur la matière cellulosique et ainsi séparée et recyclée.

8. Procédé selon les revendications 1 à 6 pour l'obtention de produits de fermentation, caractérisé en ce que, après fermentation des solutions contenant l'enzyme et les sucres, on sépare les micro-organismes du liquide contenant les produits de fermentation, on recycle le liquide contenant les produits de fermentation, en partie, en tant que diluant, dans les stades de réaction individuels, on l'évacue à nouveau de ces stades de réaction avec les produits de réaction et on le mélange en partie avec de la matière cellulosique fraîche, l'enzyme étant absorbée sur la matière cellulosique et ainsi séparée et récupérée, et en ce que les produits de fermentation sont isolés à l'état pur par distillation ou rectification du liquide les contenant.

9. Procédé selon les revendications 7 et 8, caractérisé en ce que les solutions contenant l'enzyme et les sucres ou le liquide contenant les produits de fermentation sont recyclés dans les récipients des divers stades de réaction par une alimentation au pied.

10. Appareillage pour la mise en œuvre du procédé selon les revendications 1 à 9, comprenant un dispositif de broyage (1) pour la matière première cellulosique, une unité de stérilisation (3, 5) pour la matière première broyée, un mélangeur (7) pour le mélange de la matière broyée et stérilisée avec une solution d'enzyme amenée par un conduit (8) et, le cas échéant, un récipient de fermentation (28) et un dispositif de distillation ou rectification (33), caractérisé en ce que chacun des récipients d'hydrolyse (9) — au moins deux — peut être mis en liaison par un conduit (10), d'une part, avec le mélangeur (7) et, d'autre part, avec l'évacuation de matières solides d'un dispositif de séparation (17), la partie de fond de chaque récipient d'hydrolyse (9) peut être mise en liaison par un conduit de récolte des suspensions (11) avec le dispositif de séparation (17), il y a, le cas échéant, entre le conduit de récolte (11) et le dispositif de séparation (17), dans l'ordre, un récipient équipé d'un dispositif d'agitation (12) et un broyeur (16), et l'évacuation des fractions liquides du dispositif de séparation (17) peut être mise en liaison par un conduit (22) et une alimentation (15) avec chacun des récipients d'hydrolyse (9) ainsi qu'avec le récipient de fermentation (28).